(19) **Europäisches Patentamt / European Patent Office / Office européen des brevets**

(11) **EP 4 238 540 B1**

(12) # EUROPEAN PATENT SPECIFICATION

(45) Date of publication and mention
of the grant of the patent:
**06.08.2025 Bulletin 2025/32**

(21) Application number: **21885019.6**

(22) Date of filing: **21.10.2021**

(51) International Patent Classification (IPC):
***A61F 2/966*** *(2013.01)*

(52) Cooperative Patent Classification (CPC):
**A61F 2/90; A61F 2/95;** A61F 2002/9665;
A61F 2250/001; A61F 2250/0037; A61F 2250/0039

(86) International application number:
**PCT/CN2021/125205**

(87) International publication number:
**WO 2022/089292 (05.05.2022 Gazette 2022/18)**

(54) **BEAD-STRING-SHAPED COMPONENT AND MANUFACTURING METHOD THEREFOR**

PERLENKETTENFÖRMIGE KOMPONENTE UND HERSTELLUNGSVERFAHREN DAFÜR

COMPOSANT EN FORME DE CORDON ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2020 CN 202022479731 U
30.10.2020 CN 202011195618**

(43) Date of publication of application:
**06.09.2023 Bulletin 2023/36**

(73) Proprietor: **AccuMedical Beijing Ltd.
Beijing 101399 (CN)**

(72) Inventor: **LU, Yiran
Beijing 101300 (CN)**

(74) Representative: **Elzaburu S.L.P.
Edificio Torre de Cristal
Paseo de la Castellana 259 C, planta 28
28046 Madrid (ES)**

(56) References cited:
EP-A1- 0 714 640    CN-A- 104 257 412
CN-A- 108 056 798    CN-U- 206 491 831
CN-U- 214 285 319    US-A1- 2005 131 512
US-A1- 2018 125 686    US-A1- 2019 069 903
US-B1- 6 190 393

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field

The present disclosure relates to the field of interventional therapeutics, in particular to a beaded component, a manufacturing method and application thereof.

### Background

Vascular stents (or "stents" for short) may be implanted into blood vessels via the vascular interventional procedure for the treatment of a variety of vascular diseases, for example, the blood flow guiding technique treating hemangiomas, and the vascular reconstruction technique treating stenosis or occlusion of the lumens of the blood vessels.

Some problems may be present when a vascular stent is delivered into the blood vessel. For example, upon reaching the treatment site, the stent in a compressed state is restricted from self-expansion due to being affected by torsional stresses. As another example, during stent delivery, a delivery system has poor passibility, poor vessel compliance and poor rotational coaxiality, making it difficult for the surgeon to precisely manipulate the morphology and position of the stent implant within the blood vessel during an interventional procedure.

The poorer control on the stent implant by the above delivery system reduces the stent delivery efficiency, reduces the success rate of the stent arriving at the lesion, and extends the procedure time, resulting in high risks of failure procedure and postoperative complications.

Therefore, it is necessary to propose a new solution for stent delivery.

Document US 2018/125686 discloses a stent delivery system provided comprising a radiopaque tip located at the distal end of the stent delivery system; a beads component comprising at least one expandable part; a funnel component comprising a distal flare structure and a proximal collapsed end, wherein the cross-section of the flare structure that gradually increases from its minimum diameter to its maximum diameter along the proximal-to-distal direction.

### Summary

The invention relates to a beaded component as defined by claim 1. A method of manufacturing of the component as defined by claim 7 and a stent delivery system as defined by claim 9.

The technical problems to be solved by the present disclosure are that, on the one hand, a stent cannot be released automatically due to being affected by torsional stresses,; and, on the other hand. the operation in the stent delivery process is laborious and difficult, reducing the stent delivery efficiency.

The present disclosure proposes the following technical solutions:

In a first aspect, a beaded component is provided, comprising an expandable body and at least two shaping assemblies, each shaping assembly comprises a first part and a second part;

the first part has a columnar inner cavity, and a partial section of the expandable body is bundled within the columnar inner cavity to form a shrinkage section of the beaded component; or the first part has a columnar outer surface, and an inner surface of the partial section of the expandable body is fixedly connected to the columnar outer surface to form a shrinkage section of the beaded component;
the second part is a tubular part, and the shrinkage section is sheathed in the second part;
an expansion section of the beaded component is formed between two adjacent shrinkage sections.

Preferably, the ends of the second part are provided with the chamfering structures;

preferably, the first part has a columnar inner cavity, and the first part has a shape selected from a cylinder, a prism, a sphere or a truncated cone;
preferably, the first part has a columnar outer surface;
preferably, the columnar inner cavity is cylindrical, or the columnar outer surface is cylindrical.

Preferably, the partial section of the expandable body and the columnar inner cavity are connected in a manner selected from welding, bonding, hot melt or tight fit;

preferably, the inner surface of the partial section of the expandable body and the columnar outer surface are connected in a manner selected from welding, bonding or hot melt;
preferably, the second part and the shrinkage section are connected in a manner selected from welding, bonding, hot melt or tight fit.

Preferably, the expandable body is a meshed tube, a solid-wall tube, or an expandable balloon;

preferably, the second part employs a metallic material or a polymeric material;
preferably, the second part is a heat shrink tube;
preferably, the material of the second part is selected from polyolefin material, fluorinated ethylene propylene copolymer, neoprene or fluororubber.

Preferably, a distance L between two adjacent shaping assemblies satisfies equation (1):

$$L > D \qquad (1)$$

wherein D is an outer diameter of the second part;
preferably, the expandable body comprises a first expandable body and a second expandable body. The first expandable body is sheathed in the second expandable body, and a plurality of sections of the second expandable body is bundled within the columnar inner cavity.

In a second aspect, a method manufacturing the beaded component according to any embodiment in the first aspect of the present disclosure is provided, comprising:

shrinking the expandable body;
sheathing the expandable body in at least two first parts, or placing at least two first parts inside the expandable body;
fixedly connecting the columnar inner cavities or the columnar outer surfaces of the first parts to the expandable body;
sheathing the junction of the first parts and the expandable body in the at least two second parts; and
fixedly connecting the second parts to the first parts or the expandable body to obtain the beaded component.

Preferably, the second part is heat shrink tube. The step of fixedly connecting the second parts to the first parts or the expandable body comprises:
heating the second parts to cause the second parts to be connected to the first parts or the expandable body in a hot melt manner.

In a third aspect, a stent delivery system is provided, comprising a delivery catheter, a delivery guidewire, and the beaded component according to any embodiment in the first aspect of the present disclosure;

a distal end of the delivery guidewire is fixedly connected to a proximal end of the beaded component;
the beaded component is positioned within the delivery catheter and is in a compressed state.

Preferably, the stent delivery system further comprises a stent, the stent is positioned between the delivery catheter and the beaded component and is in a compressed state;

preferably, the stent delivery system further comprises a delivery clip;
the delivery clip comprises a base and at least two clip wings. The at least two clip wings are rotationally connected to the base, respectively. The at least two clip wings, when in a first position state, jointly form a constricting part having a prismatic shape, and the constricting part has an interior space for constriction of at least a part of the stent. The at least two clip wings, when rotating from the first position state to a second position state, are separated from each other such that the constricting part expands;
the proximal end of the beaded component is fixedly sheathed in the base of the base of the delivery clip. The clip wings of the delivery clip are in the first position state and constrict at least a part of the stent therein.

The present disclosure has the following beneficial effects:
In the beaded component, the manufacturing method and application thereof provided by the present disclosure, a partial section of the expandable body is bundled within the columnar inner cavity, or the inner surface of the partial section of the expandable body is fixedly connected to the columnar outer surface such that the partial section of the expandable body shrinks to form the beaded component. When a stent is delivered using the beaded component (i.e., the beaded component is sheathed in the stent for delivery), on one hand, the expansion of the beaded component facilitates smooth release of the stent to make the stent completely fit with the lesional vessel. On the other hand, since the shaping assemblies make the partial section of the expandable body shrink, the contact area of the expandable body with the inner walls of other devices or the inner wall of the blood vessel is reduced, thereby reducing the accidental damage to the lining of the blood vessel. Meanwhile, the expansion section of the expandable body may correspondingly deform according to the course and shape of the vessel, thereby being beneficial to reducing stent delivery resistance and improving stent delivery smoothness.

Furthermore, in the beaded component, the manufacturing method and application thereof provided by the present disclosure, the beaded component may be formed on the basis of the existing expandable body (e.g., the meshed tube, the solid-wall tube, and the expandable balloon) without changing the existing manufacturing method of the expandable body, which makes the beaded component has simple structure, high manufacturability convenience and high reliability.

## Brief Description of the Drawings

In order to more clearly illustrate the technical solutions of the embodiments of the present disclosure, the drawings, which are to be used in the description of the embodiments of the present disclosure, will now be briefly introduced below. The drawings set forth herein are provided to further understand the present disclosure and constitute a part of the present disclosure. Illustrative embodiments of the present disclosure and descriptions thereof are used to explain the present disclosure and do not constitute an undue limitation of the present disclosure.

FIG. 1 is a schematic structural diagram of a beaded component in one viewing angle according to a first embodiment of the present disclosure;
FIG. 2 is a schematic structural diagram of a beaded component in another viewing angle according to a first embodiment of the present disclosure;
FIG. 3 is a schematic structural diagram of a beaded component in one viewing angle according to a second embodiment of the present disclosure;
FIG. 4 is a schematic structural diagram of a beaded component in one viewing angle according to a third embodiment of the present disclosure;
FIG. 5 is a schematic structural diagram of a beaded component in one viewing angle according to a fourth embodiment of the present disclosure;
FIG. 6 is a schematic structural diagram of a stent delivery system in one viewing angle according to a sixth embodiment of the present disclosure; and
FIG. 7 is a schematic structural diagram of a delivery clip in one viewing angle according to a sixth embodiment of the present disclosure.

Reference numerals: 1-expandable body; 1a-first expandable body; 1b-second expandable body; 2-shaping assembly; 201-first part; 202-second part; 202a-chamfering structure; 2a-first shaping assembly; 2b-second shaping assembly; 2c-third shaping assembly; 3-delivery guidewire; 4-delivery catheter; 5-stent; 6-beaded component; 8-delivery clip; 801-base; 802-clip wing; 802a-first clip wing; 802b-second clip wing.

## Detailed Description of the Embodiments

The present disclosure will now be further described in detail in conjunction with the drawings and embodiments. It should be understood that the specific embodiments described herein are merely for explaining the relevant disclosure, rather than limiting the present disclosure. Furthermore, it should be noted that the drawings only show the parts related to the relevant disclosure in order to facilitate the description.

In the description of the present disclosure, the terms "upper", "lower", "inner", "outer" and the like are only used for facilitating the description of the present disclosure and simplifying the description instead of indicating or implying that the indicated device or element has a specific orientation and is constructed and operated in the specific orientation, so that the terms cannot be understood as the limitation on the present disclosure. Further, the terms "first", "second", "third" are only for the descriptive purpose, but cannot understood as indicating or implying relative importance.

In the description of the present disclosure, it should be noted that the terms "mount", "connect", "link" and the like are understood in a broad sense, unless otherwise specified and defined., For example, it may be fixed connection, detachable connection or integrated connection; it may be mechanical connection or electrical connection; and it may be direct connection or indirect connection through intermediate media. Those skilled in the art may understand the specific meanings of the terms in the present disclosure according to specific conditions.

In the description of the present disclosure, it should be noted that the embodiments and features in the embodiments of the present disclosure may be combined with each other without conflicts.

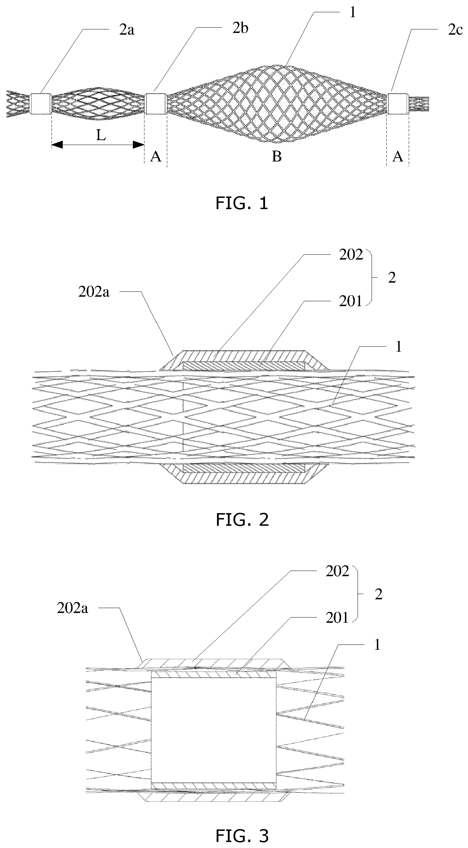

FIG. 1 is a schematic structural diagram of a beaded component in one viewing angle according to a first embodiment of the present disclosure. As shown in FIG. 1, the beaded component is divided into shrinkage sections A and expansion sections B. There are a plurality of shrinkage sections A and a plurality of expansion sections B, which are in staggered distribution. The expansion section B has a spindle-like shape, that, the expansion section B has a thicker middle and two thinner ends.

As shown in FIG. 1, the beaded component comprises an expandable body 1 and a plurality of shaping assemblies 2a, 2b and 2c. Wherein, a plurality of shaping assemblies 2a, 2b, and 2c are used to form shrinkage sections A, and an expansion section B formed between two adjacent shrinkage sections A.

FIG. 2 is a schematic structural diagram of the beaded component in another viewing angle according to the first embodiment of the present disclosure. As shown in FIG. 2, each shaping assembly comprises a first part 201. The first part 201 has a columnar inner cavity, wherein a partial section of the expandable body 1 is bundled within the columnar inner cavity.

Since the first part 201 is sectioned in FIG. 2, upper and lower halves of the first part 201 are shown in FIG. 2. It will be readily understood that in a practical structure, the upper and lower halves of the first part 201 are an integral.

In the embodiment, the first part 201 has a cylinder shape. In other embodiments, the shape of the first part 201 may also be a prism, a sphere, or a truncated cone, etc.

In the present embodiment, an inner cavity of the first part 201 is cylindrical. That is, the first part 201 is a round tubular part as a whole. In other embodiments, the inner cavity of the first part 201 may also be in prism or other shapes.

In this embodiment, the first part employs a metallic material or a polymeric material.

In the embodiment, each shaping assembly 2 further comprises a second part 202. The second part 202 is a tubular part. The shrinkage section of the beaded component is sheathed within the second part 202.

Since the second part 202 is sectioned in FIG. 2, upper and lower halves of the second part 202 are shown in FIG. 2. It will be readily understood that in a practical structure, the upper and lower halves of the second part 202 are an integral.

In the embodiment, the second part 202 employs a polymeric material. More specifically, the second part 202 is a heat shrink tube, which has the characteristics of heat shrinkage. In other embodiments, the second part 202 may also employs a metallic material.

In one example, the material of the second part is selected from a polyolefin material, fluorinated ethylene propylene copolymer, neoprene, or fluorine rubber.

In the embodiment, the second part 202 is connected to the first part 201 or the expandable body 1 in a hot melt manner. That is, the second part 202 is a heat shrink tube, which is heated after the shrinkage section is sheathed into the second part 202 such that the second part 202 is connected to the first part 201 and the expandable body 1. In other embodiments, the second part is connected to the first part or the expandable body in a manner of welding, bonding, tight fit, etc.

In the embodiment, the diameter of the columnar inner cavity of the first part 201 is smaller than the outer diameter of the expandable body 1 in an expansion state. Thus, the partial section of the expandable body 1 is bundled within the columnar inner cavity of the first part 201 in a compressed state.

In the embodiment, the partial section of the expandable body is bundled within the columnar inner cavity such that the partial section of the expandable body shrinks, thereby forming a beaded component. When a stent is delivered using the beaded component, on the one hand, the expansion of the beaded component facilitates smooth release of the stent to make the stent completely fit with the lesional vessel. On the other hand, since the shaping assemblies make the partial section of the expandable body shrink, the contact area of the expandable body with the inner walls of other devices or the inner wall of the blood vessel is reduced, thereby reducing accidental damage to the lining of the blood vessel. Meanwhile, the expansion section of the expandable body may correspondingly deform according to the course and shape of the vessel, thereby being beneficial to reducing stent delivery resistance and improving stent delivery smoothness. Disposing the second parts 202, i.e., using the shaping assemblies with a double-layer structure, further facilitates the stability and reliability of the beaded component.

Furthermore, the beaded component may be formed by using the above shaping assemblies on the basis of the existing expandable body without changing the existing manufacturing method of the expandable body, and the beaded component has simple structure, high manufacturability and high reliability.

In the embodiment in which the second part employs the heat shrink tube, the hot melt connection manner does not involve production particles caused by welding or bonding connection manner, to avoid the danger to human health by preventing the production particles from falling into the blood vessels. Furthermore, due to the wrapping of the second part on the outermost layer, the risk that the part material enters the blood vessels may be further reduced.

In the embodiment, two ends of the second part 202 both have chamfering structures 202a. The chamfering structure 202a may provide a smooth transition between the outer surface of the second part 202 and the outer surface of the expandable body 1 in a form of slope. It should be noted that the chamfering structure in the present disclosure may be any beveled structure capable of achieving the smooth transition, which may be formed in other manners besides cutting. For example, the chamfering structure may be formed by injection molding. For another example, the chamfering structure may be formed by hot molding, such as the chamfering structure 202a in FIG. 2.

It is advantageous to further reduce the resistance during stent delivery by providing the chamfering structure at the end parts of the second part. In the embodiment, three partial sections of the expandable body 1 are bundled within the columnar inner cavities of the first shaping assembly 2a, the second shaping assembly 2b, and the third shaping assembly 2c in sequence. In other embodiments, inner surfaces of a plurality of sections of the expandable body may be fixedly connected to columnar outer surfaces of a plurality of shaping assemblies.

In the embodiment, the expandable body 1 is a meshed tube. In other embodiments, the expandable body 1 may also be a solid-wall tube or an expandable balloon. Under the condition that the expandable body is an expandable balloon, the amounts of expansion of the respective expansion sections of the beaded balloon can be limited by controlling the distance between the shaping assemblies, and the delivery capability of a delivery system in an axial direction may also be improved by controlling the value of pressure charged into the balloon in combination with a beaded structure.

In the embodiment, a distance L between two adjacent shaping assemblies satisfies equation (1):

$$L > D \qquad (1)$$

wherein D is an outer diameter of the second part. As shown in FIG. 1, the distance L between two shaping assemblies is the distance between opposite end surfaces of two shaping assemblies. By designing the distance L between two adjacent shaping assemblies to satisfy equation (1), it is ensured that the diameter of each expansion section is greater than the diameter of each shrinkage section, thereby forming the beaded structure.

FIG. 3 is a schematic structural diagram of a beaded component in one viewing angle according to a second embodiment of the present disclosure. This embodiment differs from the first embodiment in that the first part 201 has a columnar outer surface, and a plurality of sections of the expandable body 1 is fixedly connected to the columnar outer surface. That is, the first part in the first embodiment is disposed outside the expandable body, while the first part in this embodiment is disposed within the expandable body. Furthermore, the second part 202 in this embodiment is disposed on the outer surface of the expandable body 1.

In the embodiment, the first part 201 has a cylindrical outer surface. Furthermore, similar to the first embodiment, the first part 201 also has a cylindrical penetrating inner cavity, i.e., the first part 201 is a circular tubular part as a whole.

In the embodiment, the inner surface of the partial section of the expandable body 1 is fixedly connected to the columnar outer surface of the first part 201 in a bonding manner. For example, the inner surface of the partial section of the expandable body 1 is coated with an adhesive and is press-fitted to the columnar outer surface of the first part 201, so that the inner surface of the partial section is fixedly connected to the columnar outer surface. Alternatively, the columnar outer surface of the first part 201 is coated with an adhesive, and the inner surface of the partial section of the expandable body 1 is press-fitted to the columnar outer surface of the first part 201 so that the inner surface of the partial section is fixedly connected to the columnar outer surface. In other embodiments, the inner surface of the partial section of the expandable body 1 and the columnar outer surface of the first part 201 can also be fixedly connected in a manner of welding, hot melt, etc.

In the embodiment, two ends of the second part 202 are likewise have chamfering structures 202a.

In the embodiment, the inner surfaces of a purity of sections of the expandable body are fixedly connected to the columnar outer surfaces of the first parts such that a purity of sections of the expandable body shrink, thereby forming a beaded component. Moreover, the second part wraps the connection region of the first part and the expandable body at the outermost layer. Thus, the beaded component in the embodiment can achieve similar technical effects as in the first embodiment, which will not be repeated herein.

Furthermore, since the first part in this embodiment is disposed within the expandable body, the first part in this embodiment can prevent further shrinkage of the expandable body at the position where the expandable body connects with the first part, which avoids detachment of the expandable body from the shaping assemblies due to the shrinkage of the expandable body when a tight fit connection is adopted, thereby improving the structure stability.

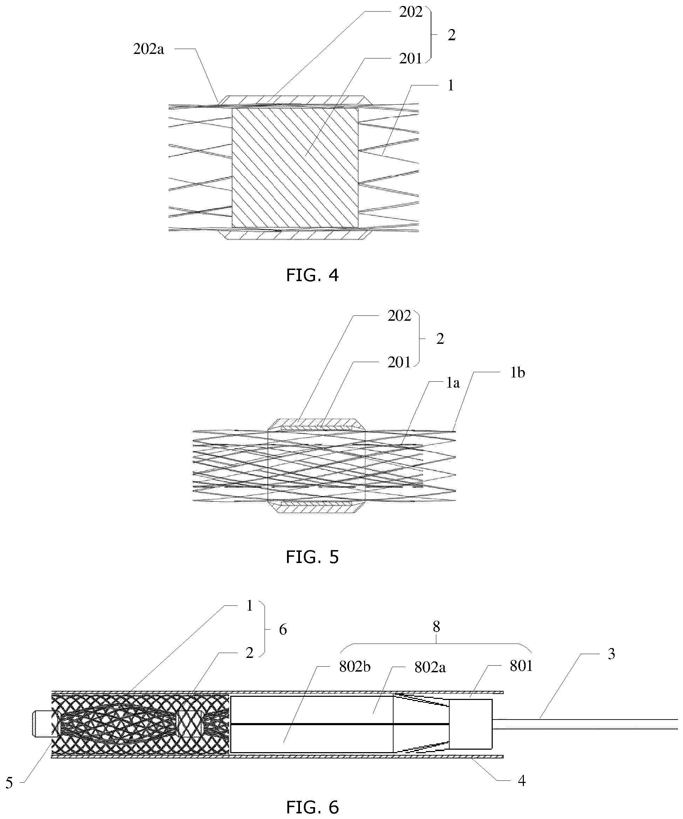

FIG. 4 is a schematic structural diagram of a beaded component in one viewing angle according to a third embodiment of the present disclosure. This embodiment differs from the second embodiment in that the first part 201 in the second embodiment has the penetrating inner cavity, while the first part 201 in this embodiment has a solid structure.

The first part in this embodiment employs the solid structure, which is advantageous in reducing the manufacturing difficulty and increasing the structural strength of the beaded component.

FIG. 5 is a schematic structural diagram of a beaded component in one viewing angle according to a fourth embodiment of the present disclosure. The beaded component in this embodiment differs from the beaded component in the first embodiment in that the beaded component in this embodiment comprises two expandable bodies, i.e., a first expandable body 1a and a second expandable body 1b.

In this embodiment, the second expandable body 1b is sheathed in the first expandable body 1a, and a partial section of the second expandable body 1b is bundled within the columnar inner cavity of the first part 201. In this way, it is advantageous to improve the bending resistance and kinking resistance of the beaded component.

A fifth embodiment of the present disclosure provides a manufacturing method of a beaded component, comprising the following steps.

First, tightening an expandable body. For example, stretch the expandable body to make the expandable body tightened.

Second, sheathing the expandable body in at least two first parts, or place at least two first parts are inside the expandable body.

Third, fixedly connecting columnar inner cavity or columnar outer surface of the first part to the expandable body. For example, a columnar inner cavity or a columnar outer surface of first part is fixedly connected to the expandable body in a manner of welding, bonding, hot melt, or tight fit.

Thereafter, sheathing the junction of the first parts and the expandable body in the at least two second parts.

Finally, fixedly connecting the second parts to the first parts or the expandable body to obtain the beaded component. For example, the first parts and the expandable body are fixedly connected to the second parts, respectively in a hot melt manner.

In some optional embodiments, the second part is a heat shrink tube. The step of fixedly connecting the second parts to the first parts or the expandable body comprises: heating the second parts to cause the second parts to be connected to the first parts or the expandable body in a hot melt manner.

FIG. 6 is a schematic structural diagram of a stent delivery system in one viewing angle according to a sixth embodiment of the present disclosure. As shown in FIG. 6, the stent delivery system comprises a delivery catheter 4, a delivery guidewire 3, and a beaded component 6, wherein the beaded component 6 comprises an expandable body 1 and a plurality of shaping assemblies 2.

In this embodiment, a distal end of the delivery guidewire 3 (i.e., the left end in FIG. 6) is fixedly connected to a proximal end of the beaded component 6 (i.e., the right end in FIG. 6). The beaded component 6 is positioned within the delivery catheter 4 and is in a compressed state.

In some optional embodiments, the stent delivery system further comprises a stent 5. The stent 5 is positioned between the delivery catheter 4 and the beaded component 6 and is in a compressed state.

In the stent delivery process, the delivery catheter 4 may firstly be delivered into the human body, and a distal opening of the delivery catheter 4 is positioned nearby a target position. Then, the delivery guidewire 3 may be pushed to deliver the beaded component 6 connected thereto distally, and the beaded component 6 drive the stent 5 to move jointly.

After the stent 5 reaches the distal opening of the delivery catheter 4, the delivery guidewire 3 continues to be pushed to gradually move the beaded component 6 and the stent 5 out of the delivery catheter 4. Due to the self-expansion characteristic of the expandable body 1, the expansion sections of the beaded component 6 which have moved out of the catheter may expand in a radial direction and drive the stent 5 in which the expansion sections sheathed to expand, so that the stent 5 is smoothly released.

In some optional embodiments, the stent delivery system further comprises a delivery clip 8.

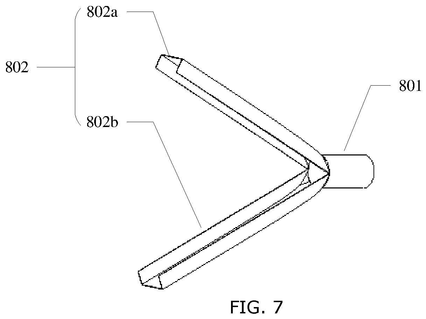

FIG. 7 is a schematic structural diagram of a delivery clip in one viewing angle according to a sixth embodiment of the present disclosure. As shown in FIG. 7, the delivery clip 8 comprises a base 801 and at least two clip wings 802. The at least two clip wings 802 are rotationally connected to the base 801, respectively. The at least two clips 802, when in a first position state, jointly form a constricting part having a prismatic shape, and the constricting part has an interior space for constricting at least a part of the stent. The at least two clip wings 802, when rotating from the first position state to a second position state, are separated from each other such that the constricting part expands.

As shown in FIG. 6, proximal end of the beaded component 6 is fixedly sheathed in the base 801 of the delivery clip 8. A first clip wing 802a and a second clip wing 802b of the delivery clip 8 are in the first position state and constrict a partial section of the stent 5 therein.

In this embodiment, the delivery clip 8 in FIG. 6 is in the first position state, and the delivery clip 8 in FIG. 7 is in the second position state.

In this embodiment, the at least two clip wings, when in the first position state, jointly form a constricting part having a prismatic shape, and the constricting part has an interior space for the constriction of at least a part of the stent. The at least two clip wings, when in the second position state, are separated such that the constricting part expands. On the one hand, the stent can be withdrawn in the event of a wrong stent release position or a need for adjustment of the stent release position. On the other hand, a stent delivery component keeps line contact with the inner wall of the delivery catheter, which reduces the contact area to reduce the frictional resistance, thereby advantageously lowering the operation difficulty and improving the delivery efficiency.

## Claims

1. A beaded component, comprising an expandable body and at least two shaping assemblies, each shaping assembly comprises a first part and a second part; wherein

the first part has a columnar inner cavity, and a partial section of the expandable body is bundled within the columnar inner cavity to form a shrinkage section of the beaded component; or the first part has a columnar outer surface, and an inner surface of the partial section of the expandable body is fixedly connected to the columnar outer surface to form a shrinkage section of the beaded component;
the second part is a tubular part, and the shrinkage section is sheathed in the second part; and
an expansion section of the beaded component is formed between two adjacent shrinkage sections.

2. The beaded component according to claim 1, wherein the end parts of the second part are provided with the chamfering structures;

preferably, the first part has a columnar inner cavity, and the first part has a shape selected from a cylinder, a prism, a sphere or a truncated cone;
preferably, the first part has a columnar outer surface; and
preferably, the columnar inner cavity is cylindrical, or the columnar outer surface is cylindrical.

3. The beaded component according to claim 1 or 2, wherein the partial section of the expandable body and the columnar inner cavity are connected in a manner selected from welding, bonding, hot melt or tight fit;

preferably, the inner surface of the partial section of the expandable body and the columnar outer surface are connected in a manner selected from welding, bonding or hot melt; and
preferably, the second part and the shrinkage section are connected in a manner selected from welding, bonding, hot melt or tight fit.

4. The beaded component according to any one of claims 1-3, wherein the expandable body is a meshed tube, a solid-wall tube, or an expandable balloon;

preferably, the second part employs a metallic material or a polymeric material;
preferably, the second part is a heat shrink tube; and
preferably, the material of the second part is selected from polyolefin material, fluorinated ethylene propylene copolymer, neoprene or fluororubber.

5. The beaded component according to any one of claims 1-4, wherein a distance L between the two adjacent shaping assemblies satisfies equation (1):

$$L > D \qquad (1)$$

wherein D is an outer diameter of the second part.

6. The beaded component according to any one of claims 1-5, wherein the expandable body comprises a first expandable body and a second expandable body, the first expandable body is sheathed in the second expandable body, and a partial section of the second expandable body is bundled within the columnar inner cavity.

7. A manufacturing method of the beaded component according to any one of claims 1-6, comprising:

shrinking the expandable body;
sheathing the expandable body in the at least two first parts, or placing the at least two first parts inside the expandable body;
fixedly connecting the columnar inner cavities or the columnar outer surfaces of the first parts to the expandable body;
sheathing the junction of the first parts and the expandable body in the at least two second parts; and
fixedly connecting the second parts to the first parts or the expandable body to obtain the beaded component.

8. The method according to claim 7, wherein the second part is a heat shrink tube; and the step of fixedly connecting the second parts to the first parts or the expandable body comprises:
heating the second parts to cause the second parts to be connected to the first parts or the expandable body in a hot melt manner.

9. A stent delivery system, comprising a delivery catheter, a delivery guidewire, and the beaded component according to any one of claims 1-6; wherein

a distal end of the delivery guidewire is fixedly connected to a proximal end of the beaded component; and the beaded component is positioned within the delivery catheter and is in a compressed state.

10. The stent delivery system according to claim 9, wherein the stent delivery system further comprises a stent, the stent is positioned between the delivery catheter and the beaded component and is in a compressed state;

preferably, the stent delivery system further comprises a delivery clip;
the delivery clip comprises a base and at least two clip wings; the at least two clip wings are rotationally connected to the base, respectively; the at least two clip wings, when in a first position state, jointly form a constricting part having a prismatic shape, and the constricting part has an interior space for the constriction of at least a part of the stent; the at least two clip wings, when rotating from the first position state to a second position state, are separated from each other such that the constricting part expands; and
the proximal end of the beaded component is fixedly sheathed in the base of the delivery clip, the clip wings of the delivery clip are in the first position state and constrict at least a part of the stent therein.

**Patentansprüche**

1. Perlenförmige Komponente, die einen expandierbaren Körper und mindestens zwei Formgebungsbaugruppen umfasst, wobei jede Formgebungsbaugruppe einen ersten Teil und einen zweiten Teil umfasst; wobei

der erste Teil einen säulenförmigen inneren Hohlraum aufweist und ein Teilabschnitt des expandierbaren Körpers innerhalb des säulenförmigen inneren Hohlraums gebündelt ist, um einen Schrumpfungsabschnitt der perlenförmigen Komponente zu bilden; oder der erste Teil eine säulenförmige Außenfläche aufweist und eine Innenfläche des Teilabschnitts des expandierbaren Körpers fest mit der säulenförmigen Außenfläche verbunden ist, um einen Schrumpfungsabschnitt der perlenförmige Komponente zu bilden;
der zweite Teil ein rohrförmiger Teil ist und der Schrumpfungsabschnitt in dem zweiten Teil ummantelt ist; und
ein Dehnungsabschnitt der perlenförmigen Komponente zwischen zwei benachbarten Schrumpfabschnitten ausgebildet ist.

2. Perlenförmige Komponente nach Anspruch 1, wobei die Endteile des zweiten Teils mit den Abschrägungsstrukturen bereitgestellt sind;

vorzugsweise weist der erste Teil einen säulenförmigen inneren Hohlraum auf und der erste Teil hat eine Form, die aus einem Zylinder, einem Prisma, einer Kugel oder einem Kegelstumpf ausgewählt ist;
vorzugsweise weist der erste Teil eine säulenförmige Außenfläche auf; und
vorzugsweise ist der säulenförmige innere Hohlraum zylindrisch oder die säulenförmige Außenfläche ist zylindrisch.

3. Perlenförmige Komponente nach Anspruch 1 oder 2, wobei der Teilabschnitt des expandierbaren Körpers und der säulenförmige innere Hohlraum auf eine Weise verbunden sind, die aus Schweißen, Kleben, Heißschmelzen oder Presspassung ausgewählt ist;

vorzugsweise sind die Innenfläche des Teilabschnitts des expandierbaren Körpers und die säulenförmige Außenfläche auf eine Weise verbunden, die aus Schweißen, Kleben oder Heißschmelzen ausgewählt ist; und
vorzugsweise sind der zweite Teil und der Schrumpfabschnitt in einer Weise verbunden, die aus Schweißen, Kleben, Heißschmelzen oder Presspassung ausgewählt ist.

4. Perlenförmige Komponente nach einem der Ansprüche 1 bis 3, wobei der expandierbare Körper ein Netzschlauch, ein Vollwandschlauch oder ein expandierbarer Ballon ist;

vorzugsweise verwendet der zweite Teil ein metallisches Material oder ein polymeres Material;
vorzugsweise ist der zweite Teil ein Wärmeschrumpfschlauch; und

vorzugsweise ist das Material des zweiten Teils aus Polyolefinmaterial, fluoriertem Ethylen-Propylen-Copolymer, Neopren oder Fluorkautschuk ausgewählt.

5. Perlenförmige Komponente nach einem der Ansprüche 1 bis 4, wobei ein Abstand L zwischen den zwei benachbarten Formgebungsbaugruppen die Gleichung (1) erfüllt:

$$L > D \qquad\qquad (1)$$

wobei D ein Außendurchmesser des zweiten Teils ist.

6. Perlenförmige Komponente nach einem der Ansprüche 1 bis 5, wobei der expandierbare Körper einen ersten expandierbaren Körper und einen zweiten expandierbaren Körper umfasst, der erste expandierbare Körper von dem zweiten expandierbaren Körper ummantelt ist und ein Teilabschnitt des zweiten expandierbaren Körpers innerhalb des säulenförmigen inneren Hohlraums gebündelt ist.

7. Herstellungsverfahren der Perlenförmige Komponente nach einem der Ansprüche 1 bis 6, umfassend:

Schrumpfen des expandierbaren Körpers;
Umhüllen des expandierbaren Körpers in den mindestens zwei ersten Teilen oder Anordnen der mindestens zwei ersten Teile innerhalb des expandierbaren Körpers;
festes Verbinden der säulenförmigen inneren Hohlräume oder der säulenförmigen Außenflächen der ersten Teile mit dem expandierbaren Körper;
Umhüllen der Verbindung der ersten Teile und des expandierbaren Körpers mit den mindestens zwei zweiten Teilen; und
festes Verbinden der zweiten Teile mit den ersten Teilen oder dem expandierbaren Körper, um die perlenförmige Komponente zu erhalten.

8. Verfahren nach Anspruch 7, wobei der zweite Teil ein Wärmeschrumpfschlauch ist; und der Schritt des festen Verbindens der zweiten Teile mit den ersten Teilen oder dem expandierbaren Körper Folgendes umfasst:
Erwärmen der zweiten Teile, um zu bewirken, dass die zweiten Teile heißschmelzend mit den ersten Teilen oder dem expandierbaren Körper verbunden werden.

9. Stentzuführsystem, umfassend einen Zuführkatheter, einen Zuführdraht und die perlenförmige Komponente nach einem der Ansprüche 1 bis 6; wobei

ein distales Ende des Zuführdrahts fest mit einem proximalen Ende der perlenförmigen Komponente verbunden ist; und
die perlenförmige Komponente innerhalb des Zuführkatheters positioniert ist und sich in einem komprimierten Zustand befindet.

10. Stentzuführsystem nach Anspruch 9, wobei das Stentzuführsystem ferner einen Stent umfasst, wobei der Stent zwischen dem Zuführkatheter und der perlenförmigen Komponente positioniert ist und sich in einem komprimierten Zustand befindet;

vorzugsweise umfasst das Stentzuführsystem ferner einen Zuführclip;
der Zuführclip umfasst eine Basis und mindestens zwei Clipflügel; die mindestens zwei Clipflügel sind jeweils drehbar mit der Basis verbunden; die mindestens zwei Clipflügel in einem ersten Positionszustand bilden gemeinsam einen Einschnürungsteil mit einer prismatischen Form und der Einschnürungsteil weist einen Innenraum für die Einschnürung mindestens eines Teils des Stents auf; die mindestens zwei Clipflügel sind beim Drehen aus dem ersten Positionszustand in einen zweiten Positionszustand voneinander getrennt, so dass sich der Einschnürungsteil ausdehnt; und
das proximale Ende der perlenförmigen Komponente ist in der Basis des Zuführclips fest ummantelt, die Clipflügel des Zuführclips befinden sich in dem ersten Positionszustand und verengen mindestens einen Teil des Stents darin.

**Revendications**

1. Un composant perlé, comprenant un corps extensible et au moins deux ensembles de mise en forme, chaque ensemble de mise en forme comprenant une première partie et une deuxième partie ; dans lequel

   la première partie a une cavité intérieure colonnaire, et une section partielle du corps expansible est regroupée à l'intérieur de la cavité intérieure colonnaire pour former une section de retrait du composant perlé ; ou la première partie a une surface extérieure colonnaire, et une surface intérieure de la section partielle du corps expansible est reliée de manière fixe à la surface extérieure colonnaire pour former une section de retrait du composant perlé ; la deuxième partie est une partie tubulaire, et la section de retrait est gainée dans la deuxième partie ; et une section d'expansion du composant perlé est formée entre deux sections de retrait adjacentes.

2. Le composant perlé selon la revendication 1, dans lequel les parties d'extrémité de la seconde partie sont pourvues des structures de chanfreinage ;

   de préférence, la première partie a une cavité interne colonnaire, et la première partie a une forme choisie parmi un cylindre, un prisme, une sphère ou un cône tronqué ;
   de préférence, la première partie présente une surface extérieure colonnaire ; et
   de préférence, la cavité interne colonnaire est cylindrique, ou la surface externe colonnaire est cylindrique.

3. Le composant perlé selon la revendication 1 ou 2, dans lequel la section partielle du corps expansible et la cavité interne colonnaire sont connectées d'une manière choisie entre le soudage, le collage, le thermofusible ou l'ajustement serré ;

   de préférence, la surface interne de la section partielle du corps expansible et la surface externe en colonne sont reliées d'une manière choisie entre le soudage, le collage ou le thermofusible ; et
   de préférence, la deuxième partie et la section de rétrécissement sont reliées d'une manière choisie entre le soudage, le collage, le thermofusible ou l'ajustement serré.

4. Le composant perlé selon l'une quelconque des revendications 1 à 3, dans lequel le corps expansible est un tube maillé, un tube à paroi solide ou un ballonnet expansible ;

   de préférence, la deuxième partie utilise un matériau métallique ou un matériau polymère ; de préférence, la deuxième partie est un tube thermorétractable ; et
   de préférence, le matériau de la deuxième partie est choisi entre un matériau polyoléfinique, un copolymère d'éthylène propylène fluoré, du néoprène ou du caoutchouc fluoré.

5. Le composant perlé selon l'une quelconque des revendications 1 à 4, dans lequel une distance L entre les deux ensembles de mise en forme adjacents satisfait à l'équation (1) :

$$L > D \qquad\qquad\qquad\qquad (1)$$

   dans laquelle D est un diamètre extérieur de la deuxième partie.

6. Le composant perlé selon l'une quelconque des revendications 1 à 5, dans lequel le corps expansible comprend un premier corps expansible et un second corps expansible, le premier corps expansible étant gainé dans le second corps expansible, et une section partielle du second corps expansible est groupée à l'intérieur de la cavité interne colonnaire.

7. Un procédé de fabrication du composant perlé selon l'une quelconque des revendications 1 à 6, comprenant :

   le rétrécissement du corps expansible ;
   le revêtement du corps expansible au moins dans les deux premières parties, ou le placement au moins dans les deux premières parties à l'intérieur du corps expansible ;
   la connexion de de manière fixe des cavités intérieures colonnaires ou des surfaces extérieures colonnaires des premières parties au corps expansible ;
   le gainage de la jonction des premières parties et du corps expansible au moins dans les deux secondes parties ;

et
la connexion de manière fixe des secondes parties aux premières parties ou au corps expansible pour obtenir le composant perlé.

8. Le procédé selon la revendication 7, dans lequel la seconde partie est un tube thermorétractable, et l'étape de connexion fixe des secondes parties aux premières parties ou au corps expansible comprend :
le chauffage des secondes parties pour amener les secondes parties à être connectées aux premières parties ou au corps expansible d'une manière thermofusible.

9. Un système de pose d'endoprothèse, comprenant un cathéter de pose, un fil-guide de pose et le composant perlé selon l'une quelconque des revendications 1 à 6 ; dans lequel :

une extrémité distale du fil-guide de distribution est connectée de manière fixe à une extrémité proximale du composant perlé ; et
le composant perlé est positionné à l'intérieur du cathéter d'administration et est dans un état comprimé.

10. Le système de pose d'endoprothèse selon la revendication 9, dans lequel le système de pose d'endoprothèse comprend en outre une endoprothèse, l'endoprothèse est positionnée entre le cathéter de pose et le composant perlé et est dans un état comprimé ;

de préférence, le système de mise en place d'endoprothèse comprend en outre une pince de mise en place ; l'agrafe de distribution comprend une base et au moins deux ailes d'agrafe ; deux ailes d'agrafe au moins sont connectées en rotation à la base, respectivement ; deux ailes d'agrafe au moins, lorsqu'elles sont dans un premier état de position, forment conjointement une partie de constriction ayant une forme prismatique, et la partie de constriction a un espace intérieur pour la constriction d'au moins une partie de l'endoprothèse ; deux ailes d'agrafe au moins, lorsqu'elles tournent du premier état de position à un second état de position, sont séparées l'une de l'autre, de sorte que la partie de constriction se dilate ; et
l'extrémité proximale du composant perlé est gainée de manière fixe dans la base de l'agrafe de distribution, les ailes de l'agrafe de distribution sont dans le premier état de position et rétrécissent au moins une partie de l'endoprothèse.

FIG. 1

FIG. 2

FIG. 3

FIG. 4

FIG. 5

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018125686 A